(19) Europäisches Patentamt
European Patent Office
Office européen des brevets

(11) EP 3 687 983 B1

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(21) Anmeldenummer: **18779609.9**

(22) Anmeldetag: **24.09.2018**

(51) Internationale Patentklassifikation (IPC):
***C07D 303/22*** *(2006.01)*

(52) Gemeinsame Patentklassifikation (CPC):
**C07D 303/22**

(86) Internationale Anmeldenummer:
**PCT/EP2018/075730**

(87) Internationale Veröffentlichungsnummer:
**WO 2019/063454 (04.04.2019 Gazette 2019/14)**

(54) **FLUORVERBINDUNGEN**

FLUORINE COMPOUNDS

COMPOSÉS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.09.2017 EP 17193042**

(43) Veröffentlichungstag der Anmeldung:
**05.08.2020 Patentblatt 2020/32**

(73) Patentinhaber: **Merck Patent GmbH**
**64293 Darmstadt (DE)**

(72) Erfinder:
- **FRIEDRICH, Reiner**
**64342 Seeheim-Jugenheim (DE)**
- **KOCH, Fabian**
**64319 Pfungstadt (DE)**

(56) Entgegenhaltungen:
**WO-A1-2015/124290 WO-A1-2016/096128**
**WO-A1-2016/096129 WO-A1-2017/216201**

- **SOLOV'EV, D. V.; KOLOMENSKAYA, L. V.; RODIN, A. A.; ZENKEVICH, I. G.; LAVRENT'EV, A. N.: "Fluorine-containing glycidyl ethers. Synthesis and spectra", ZHURNAL OBSHCHEI KHIMII, Bd. 61, Nr. 3, 1. Januar 1991 (1991-01-01) , Seiten 673-679, XP009509482, ISSN: 0044-460X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 687 983 B1

## Beschreibung

Gegenstand der vorliegenden Erfindung sind Fluorverbindungen, Verfahren zu deren Herstellung und deren Verwendung.

Fluorierte Verbindungen sind seit langem Bestandteil in Polymeren für schmutzabweisende Beschichtungen. Klassische Fluorverbindungen sind aus langkettigen, perfluorierten Alkylketten (C6-C8) aufgebaut und gelten potentiell als bioakkumulativ und toxisch. Problematisch sind bei herkömmlicher C6-Verbindungen deren Persistenz und die damit verbundenen Umweltgefahr. Fluorhaltige Verbindungen werden auch als Grenzflächenvermittler bzw. Emulgator oder Viskositätsminderer in Farben, Lacken oder Klebstoffen verwendet.

Bisherige Bemühungen zielten darauf ab, Verbindungen zu untersuchen, die kurzkettig sind und eine Sollbruchstelle besitzen, wodurch eine möglichst vollständige Zersetzung (Mineralisierung) der fluorierten Einheiten gewährleistet werden soll. Kürzerkettige Fluorbausteine sind von ihren ökotoxikologischen Profilen günstiger, zeigen jedoch in ihren Anwendungsbereichen oft schlechtere Eigenschaften.

Möglichst kurzkettige Fluorbausteine sind auf der einen Seite hilfreich, wenn es darum geht nicht-persistenten Verbindungen in die Umwelt zu emittieren. Auf der anderen Seite ist der Fluoranteil entscheidend für die Schmutzabweisung. Je weniger Fluor im Molekül, desto schlechter der Effekt. Daher wäre es wünschenswert aus kurzkettigen fluorhaltigen Moleküle (Monomere) funktionalisierbare Makromoleküle mit mehreren Wiederholungseinheiten zu synthetisieren. Es besteht daher Bedarf an neuen fluorhaltigen Verbindungen und an Verfahren zur Herstellung dieser Verbindungen.

WO 2016/096128 A1, WO 2016/069129 A1, WO 2015/124290 A1 und Solov'ev et al., Zhurnal Obshchei Khimii 1991, 61(3), 673-679 beschreiben Verbindungen mit fluorierten Endgruppen, offenbaren aber keine Verbindungen wie in der vorliegenden Anmeldung beschrieben und beansprucht und legen diese auch nicht nahe.

Gegenstand der vorliegenden Erfindung sind Verbindungen der Formeln (I), (II) und (III), Verfahren zu deren Herstellung, die Verwendung dieser Verbindungen zur Herstellung weiterer fluorierter Verbindungen, fluorierte Verbindungen der Formeln (XI) bis (XVI) und deren Verwendungen und Mittel wie nachfolgend beschrieben.

Ein erster Gegenstand der Erfindung sind Verbindungen der Formeln (I), (II) und (III):

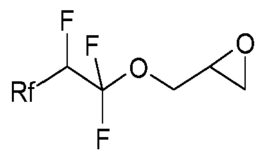

(I)

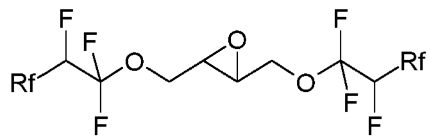

(II)

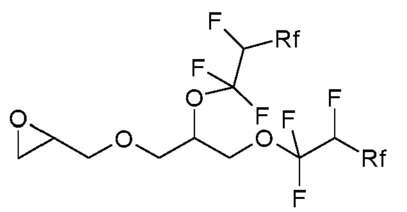

(III)

wobei
Rf eine Gruppe der Formel $CF_3-(CF_2)_a-O_b-(CF_2)_c-O_d-$ ist,
wobei

a = 1, 2 oder 3,
b = 0 oder 1,
c = 0, 1, 2 oder 3 und
d = 0 oder 1 ist.

Besonders bevorzugt ist Rf eine Gruppe der Formel $CF_3$-$(CF_2)_{1-3}$- oder der Formel $CF_3$-$(CF_2)_{1-3}$-O-.

Ein weiterer Gegenstand der Erfindung ist ein Verfahren zur Herstellung von Verbindungen der Formel (I) durch Umsetzen von Verbindungen der Formel (IV) mit Glycidol (V) zu Verbindungen der Formel (I)

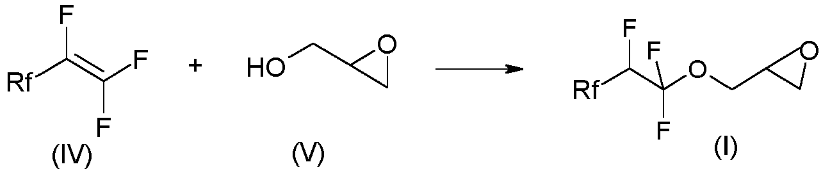

(IV) (V) (I)

Dazu wird das entsprechende Perfluoroolefin mit einer Base, bevorzugt ein Alkalicarbonat oder -hydroxid, in einem organischen Lösungsmittel, bevorzugt einem Ether wie Dioxan, THF oder aber auch Acetonitril, in einem Druckreaktor bei erhöhter Temperatur, bevorzugt 80-120°C, für mehrere Stunden zur Reaktion gebracht, bevorzugt 6-16 Stunden. Das Epoxid fällt dabei in guter Ausbeute an (70-95%)

Ein weiterer Gegenstand der Erfindung ist ein Verfahren zur Herstellung von Verbindungen der Formel (II) durch Umsetzen von Verbindungen der Formel (IV) mit Verbindungen der Formel (VI) zu Verbindungen der Formel (II)

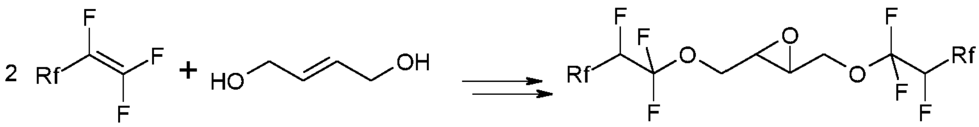

(IV) (VI) (II)

Ein weiterer Gegenstand der Erfindung ist ein Verfahren zur Herstellung von Verbindungen der Formel (III) durch Umsetzen von Verbindungen der Formel (IV) mit Verbindungen der Formel (VII) zu Verbindungen der Formel (II)

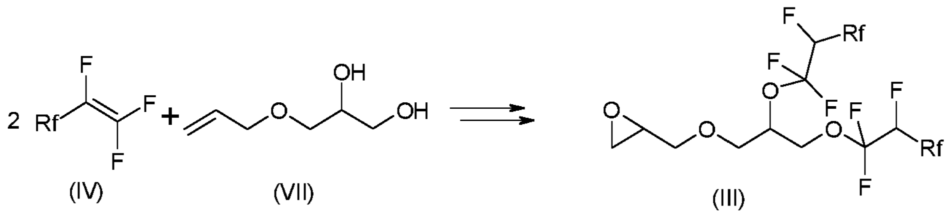

(IV) (VII) (III)

Die Gruppe Rf hat in den oben genannten Verfahren die für die Verbindungen (I), (II) und (III) angegebene Bedeutung, insbesondere auch die bevorzugte Bedeutung.

Auch bei den Verfahren der Umsetzung der Perfluorolefine mit den oben erwähnten Dihydroxyalkylolefinen, hat es sich bewährt, mit einer Base, bevorzugt ein Alkalicarbonat oder -hydroxid als Base, in einem organischen Lösungsmittel, bevorzugt einem Ether wie Dioxan, THF oder aber auch Acetonitril, die Reaktion in einem Druckreaktor bei erhöhter Temperatur, bevorzugt 80-120°C, für mehrere Stunden (bevorzugt 6-16 Stunden) durchzuführen, um das Produkt in guter Ausbeute (70-95%) zu erhalten. Anschließend wird hier die Doppelbindung unter bekannten Epoxidierungsbedingungen zum entsprechenden Monomer umgesetzte. Besonders bewährt hat sich hier die Umsetzung mit m-Chlorperbenzoesäure oder die Epoxidierung nach Jacobsen.

Durch die vorliegende Erfindung ist es nun möglich ausgehend von den Verbindungen der Formeln (I), (II) und (III) durch Polymerisation nach bekannten Methoden entsprechende Makromoleküle der Formeln (VIII) bis (X) herzustellen:

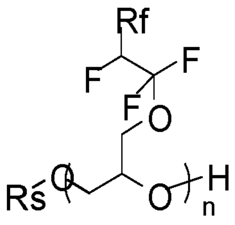

(VIII)

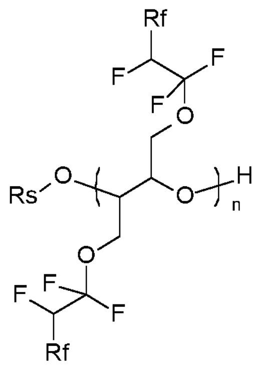

(IX)

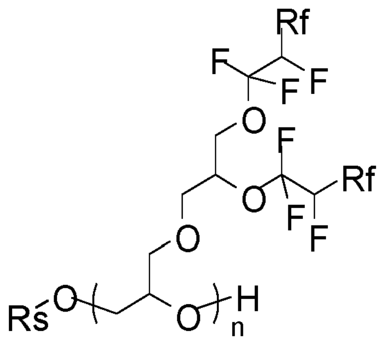

(X)

wobei

die Gruppe Rf die für die Verbindungen (I), (II) und (III) angegebene Bedeutung hat und n gleich 5 bis 20 ist. Rs ist hierbei die gegebenenfalls fluorierte Alkylgruppe eines "Starter" Alkohol, der ebenfalls fluoriert sein kann. Bevorzugt hat Rs die folgende Struktur:
Rs:

Rf' $-CHF-CF_2-CH_2-$

Die Synthese der Alkohole ist z.B. in J.D.LaZerte, R.J. Koshar Journal of the American Chemical Society 1955 77 910-914 beschrieben.

Bevorzugt ist Rf' eine Gruppe der Formel $CF_3-(CF_2)_a-O_b-(CF_2)_c-O_d-$, wobei

a = 1, 2 oder 3,
b = 0 oder 1,
c = 0, 1, 2 oder 3 und
d = 0 oder 1 ist.

Besonders bevorzugt ist Rf' eine Gruppe der Formel $CF_3-(CF_2)_{1-3}-$ oder der Formel $CF_3-(CF_2)_{1-3}-O-$.
Diese "Kamm-"Polymere der Formeln (VIII) bis (X) besitzen jeweils eine terminale OH Gruppe die weiter umgesetzt werden kann. Für schmutzabweisende Beschichtungen ist es vorteilhaft dort (Meth)acrylgruppe zu verestern. Diese Monomere der Formeln (XI), (XIII) und (XV) können dann wiederum zu Copolymeren umgesetzt werden, die auf die genauen Bedürfnisse der Textilfaser abgestimmt sind.

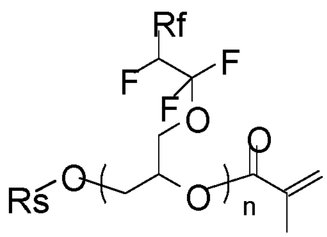

(XI)

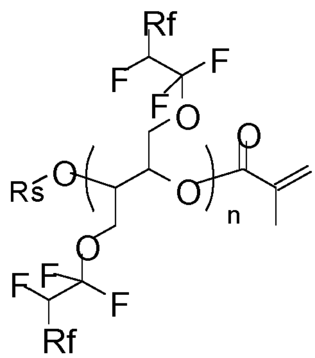

(XIII)

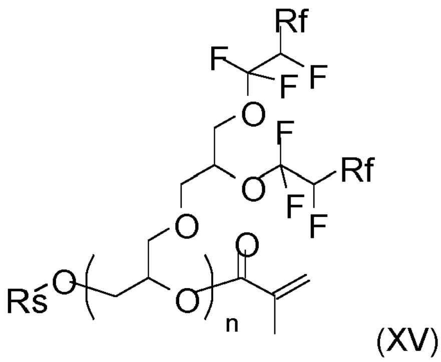

(XV)

wobei
die Gruppe Rf die für die Verbindungen (I), (II) und (III) angegebene Bedeutung hat und n gleich 5 bis 20 ist. Die Gruppe Rs hat die für die Verbindungen der Formeln (VIII)-(X) angegebene Bedeutung, insbesondere auch die bevorzugte Bedeutung.

Die Umsetzung zu den (Meth)acrylaten erfolgt hier vorteilhaft ausgehend von den Säurechloriden bzw. -anhydriden nach dem Fachmann bekannten Methoden.

Darüber hinaus lässt sich an der endständigen OH-Gruppe der "Kamm-"Polymere der Formeln (VIII) bis (X) auch weiter Ethylenoxid anlagern. So gelangt man zu nichtionischen Tensiden der Formeln (XII), (XIV) und (XVI) mit fluorierten Seitengruppen.

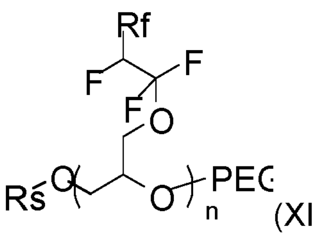

(XII)

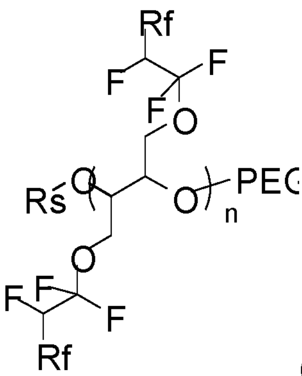

(XIV)

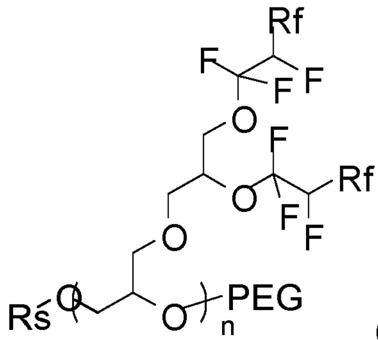

(XVI)

wobei

die Gruppe Rf die für die Verbindungen (I), (II) und (III) angegebene Bedeutung hat und n gleich 5 bis 20 ist. Die Gruppe Rs hat die für die Verbindungen der Formeln (VIII)-(X) angegebene Bedeutung, insbesondere auch die bevorzugte Bedeutung.

Vorteilhaft bei den Verbindungen XII, XIV und XVI ist, dass zu ihrer Herstellung die weitere Umsetzung der "Kamm"-Polymere der Formeln (VIII) bis (X) mit Ethylenoxid zum nichtionischen Tensid in einem Ansatz gefahren werden kann, da die Reaktionsbedingungen (Katalysator und Temperatur) mit der Polymerisation der fluorierten Epoxide identisch ist.

Bevorzugt können die erfindungsgemäßen Verbindungen eine besondere Oberflächenaktivität aufweisen. Die erfindungsgemäßen Verbindungen, insbesondere die bevorzugten Verbindungen, können außerdem verbesserte Umwelteigenschaften aufweisen, da sie weder chemisch noch biologisch zu langkettigen PFCAs oder PFASs abbauen. Bevorzugt können die erfindungsgemäßen Verbindungen durch entsprechende Umwelteinflüsse vollständig in mineralisierbare/regenerierbare Verbindungen überführt werden.

Die Verwendung von Verbindungen der Formeln (VIII) bis (XVI) und Mittel enthaltend diese Verbindungen sind ebenfalls Gegenstand der Erfindung.

Die erfindungsgemäßen Verbindungen können alleine oder als Gemisch, auch mit anderen fluorierten und/oder nicht fluorierten Verbindungen, verwendet werden, insbesondere zur Herstellung von funktionellen Überzügen und Oberflächenmodifikationen aller Art auf Gegenständen sowohl für Innen- wie auch für Außenbereiche. Prinzipiell können alle Oberflächen beschichtet werden, insbesondere Glas, Keramik, Emaille, Metalle, Kunststoffe, Elastomere, Naturstoffe, Textilien, gegebenenfalls nach einer geeigneten Vorbehandlung.

Neben den Verbindungen der Erfindung können die Beschichtungen auch Lösemittel, Additive, Tenside, Hilfs- und Füllstoffe enthalten. Beispielhaft seien auch Silikonpartikel und, ggf. oberflächenmodifizierte, Pigmente genannt.

Bevorzugte Einsatzgebiete sind beispielsweise die Verwendung der erfindungsgemäßen Verbindungen in Beschichtungen für optische Elemente oder Textilien, wie z. B. die Verwendung in Antifingerprint Coatings, z. B. für Displays, optische Linsen, Brillengläser, Objektive für Kameras, Ferngläser, Fensterscheiben oder Spiegel, oder als Hydrophobiermittel zur Textilausrüstung.

Die Aufbringung der erfindungsgemäßen Verbindungen oder sie enthaltenden Mischungen auf eine geeignete Oberfläche kann, vollflächig oder teilflächig, durch verschiedene dem Fachmann bekannte Beschichtungsprozesse erfolgen, z. B. mittels CVD-, PVD-, Spray-Coating-, Ink-Jet-, Offset-Prozessen.

Gegenstand der Erfindung sind auch Mittel, in denen mindestens eine der erfindungsgemäßen Verbindungen enthalten ist, wobei die Mittel auch Lösemittel, Additive, Tenside, Hilfs- und Füllstoffe enthalten können

Gegenstand der Erfindung sind auch beschichtete Gegenstände, insbesondere die vorstehend genannten Gegenstände, deren Beschichtung unter Verwendung von mindestens einer erfindungsgemäßen Verbindung hergestellt wurde. Bevorzugt sind Displays, optische Linsen, Brillengläser, Objektive für Kameras, Ferngläser, Fensterscheiben, Spiegel und Textilien.

Die Verbindungen der Formeln (VIII) bis (X) und (XII), (XIV) und (XVI) können bevorzugt als oberflächenaktive Mittel verwendet werden, bevorzugt als Tensid, Hydrophobiermittel, Grenzflächenvermittler, Viskositätsminderer, Schaumstabilisator oder Emulgator. Ein weiterer Gegenstand der vorliegenden Erfindung ist somit die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen, insbesondere der genannten besonders bevorzugten Verbindungen.

Neben diesen Verbindungen können die erfindungsgemäßen Mischungen auch Lösemittel, Additive, Hilfs- und Füllstoffe sowie nicht fluorierte Tenside enthalten. Beispielhaft seien genannt Silikonpartikel, Weichmacher und oberflächenmodifizierte Pigmente.

Bevorzugte Einsatzgebiete sind beispielsweise die Verwendung der erfindungsgemäßen Fluortenside als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen, in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen. Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung.

Entsprechende Mittel, enthaltend die erfindungsgemäßen Fluorverbindungen, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Lackformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside, insbesondere der bevorzugten Verbindungen, sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die folgenden Beispiele erläutern die vorliegende Erfindung näher, ohne den Schutzbereich zu beschränken.

## Beispiele

Die NMR-Spektren werden mit einem Bruker 400 MHz Spektrometer mit internem Standard gemessen.

Die IR-Spektren werden mit einem Brucker Alpha Platinum-ATR Spektrometer gemessen.

**Bestimmung der statischen Oberflächenspannung**

Es werden die statischen Oberflächenspannungen $\gamma$ von wässrigen Tensidlösungen mit verschiedenen Konzentrationen c (Gramm pro Liter) bestimmt.

Gerät: Tensiometer der Firma Dataphysics (Modell DCAT 11)
Temperatur der Messlösungen: 20°±0,2°C
Eingesetzte Messmethode: Messung der Oberflächenspannung mit der Wilhelmy Plattenmethode nach DIN EN 14370.
Platte: Platin, Länge= 19,9mm

Bei der Plattenmethode wird die Oberflächen- bzw. Grenzflächenspannung der Tensidlösung aus der auf die benetzte Länge einer Platte wirkenden Kraft nach folgender Formel berechnet: $\gamma = \frac{F}{L \cdot \cos\theta} = \frac{F}{L}$ $\gamma$= Grenz- oder Oberflächenspannung; F= auf die Waage wirkende Kraft; L= benetzte Länge (19,9 mm); $\theta$= Kontaktwinkel)Die Platte besteht aus angerautem Platin und wird also optimal benetzt, so dass der Kontaktwinkel $\theta$ nahe bei 0° liegt. Der Term cos $\theta$ erreicht daher annähernd den Wert 1, so dass nur noch die gemessene Kraft und die Länge der Platte berücksichtigt werden müssen.

**Abkürzungen**

| | |
|---|---|
| EO | Ethylenoxideinheiten |
| THF | Tetrahydrofuran |
| MTBE | tert-Butylmethylether |
| PPVE | Perfluorpropylvinylether |
| Sdp. | Siedepunkt |
| w% | Gewichtsprozent |

**Beispiel 1:**

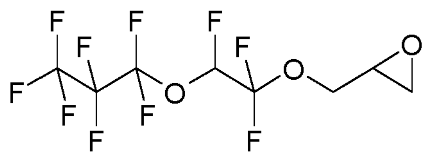

21.78 g Glycidol, 93,86 g PPVE, 12,19 g Kaliumcarbonat; 130 ml Dioxan Die Edukte werden in einem 300 mL-Druckreaktor zusammengegeben und für 24h bei 110C gerührt. Zu Beginn der Reaktion stellt sich ein Druck von 4,5bar ein, dieser fällt über Nacht auf 0,5bar ab. Das Reaktionsgemisch wird mit Wasser und MTBE versetzt und die Phasen getrennt. Die wässrige Phase wird mit 2x30mL MTBE extrahiert und die vereinigte organische Phase mit 40 mL Wasser und 40 mL gesättigter NaCl-Lösung gewaschen. Das Extrakt wird über Natriumsulfat getrocknet und das Lösemittel abdestilliert. Auswaage: 90,87 g Das Produkt wurde im Vakuum destilliert.

| $T_{Bad}$ °C | $T_{Kopf}$ °C | p mbar |
|---|---|---|
| 50,3 | 24,1 | 0,80 |

**Beispiel 2:**

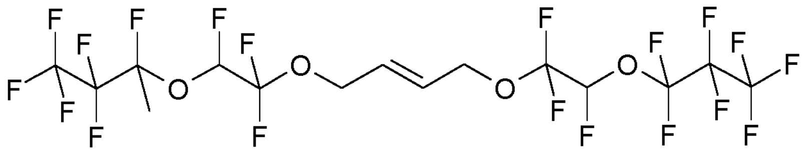

10,5 g 1,4-Butendiol und 80,0 g PPVE 2,01 g KOH und 40 g Acetonitril werden zusammen in einen Druckreaktor gegeben und das Gemisch auf 80°C erhitzt. Nach 16 Stunden ist die Reaktion beendet. Das Reaktionsgemisch wird mit Wasser und MTBE versetzt und die Phasen getrennt. Die wässrige Phase wird mit 2x30mL MTBE extrahiert. Die vereinigte organische Phase wird mit 50mL Wasser und 50mL gesättigter NaCl-Lösung gewaschen. Das Extrakt wird über Natriumsulfat getrocknet. Das Lösemittel wird im Vakuum entfernt. Ausbeute: m=56,26g

$^{1}$H-NMR: 7,2ppm (dt, 2 H, -CFH); 5,9ppm (t, 1,5H, -CH=CH-); 5,7ppm (t, 0,5H, -CH=CH-) 4,6 ppm (d, 1H, -CH$_2$-O); 4,5 ppm (d, 3H, -CH$_2$-O);
1H-NMR: 7,2 ppm (d, 1H, -CH$_2$-O); 4,5 ppm (d, 3H, -CH$_2$-O);

**Beispiel 3:**

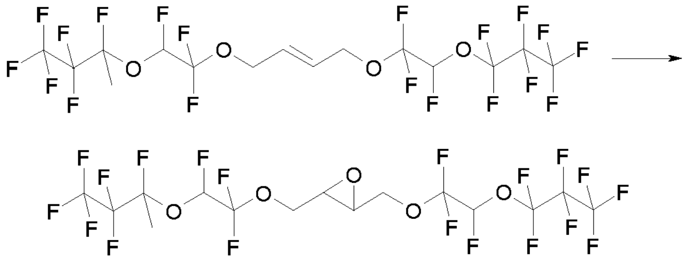

In einem Dreihalskolben wird unter Schutzgasatmosphäre 10 g des Olefins aus Beispiel 2 in 40 ml Acetonitril vorgelegt. Anschließend wird unter Kühlung 7 g m-Chlorperbenzoesäure zugegeben und das Reaktionsgemisch für 20 Stunden bei 80°C gerührt. Das Reaktionsgemisch wird mit Wasser und MTBE versetzt und die Phasen getrennt. Die wässrige Phase wird mit 2x30mL MTBE extrahiert. Danach wird die vereinigte organische Phase mit jeweils 40mL Wasser und 40mL gesättigter NaCl-Lösung gewaschen. Anschließend wird das Extrakt über Natriumsulfat getrocknet und das Lösemittel abdestilliert. Es bleibt ein weißer Feststoff als Rückstand. Auswaage: 15,22g

**Beispiel 4:**

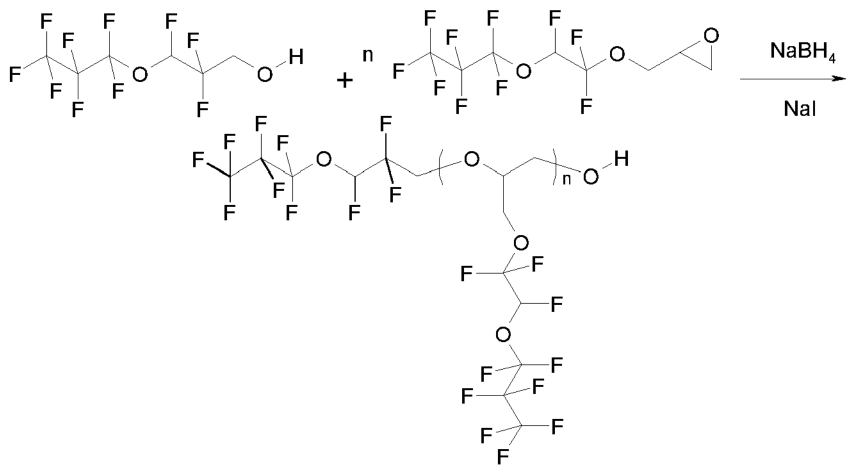


In einem Stahlautoklaven wird das in Beispiel 1 hergestellte Epoxid mit $NaBH_4$ und NaI Komplex als Starter

bei 120 °C zu dem entsprechendem PEG umgesetzt. Über Startermenge und Reaktionszeit/-temperatur kann das Molekulargewicht eingestellt werden.

## Patentansprüche

**1.** Verbindungen der Formel (I), (II) oder (III)

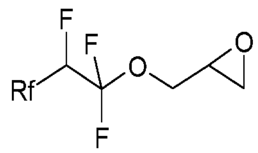

(I)

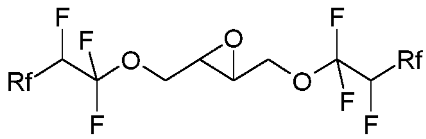

(II)

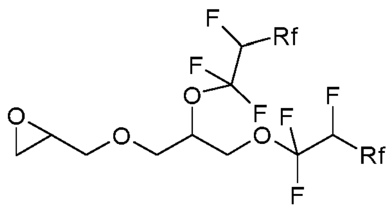

(III)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist.

**2.** Verbindungen gemäß Anspruch 1, **dadurch gekennzeichnet, dass** Rf eine Gruppe der Formel $CF_3-(CF_2)_{1-3}-$ oder $CF_3-(CF_2)_{1-3}-O-$ ist.

**3.** Verfahren zur Herstellung von Verbindungen der Formel (I)

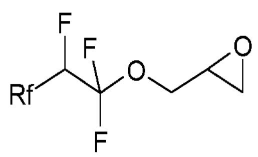

(I)

durch Umsetzen von Verbindungen der Formel (IV) mit Glycidol (V) zu Verbindungen der Formel (II)

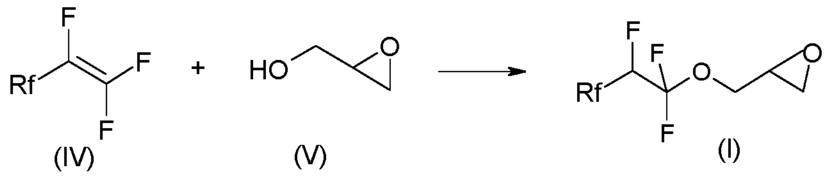

(IV) (V) (I)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist.

**4.** Verfahren zur Herstellung von Verbindungen der Formel (II)

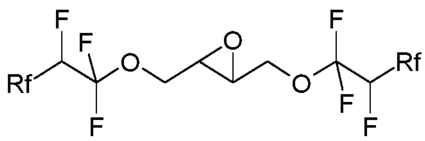

(II)

durch Umsetzen von Verbindungen der Formel (IV) mit Verbindungen der Formel (VI) zu Verbindungen der Formel (II)

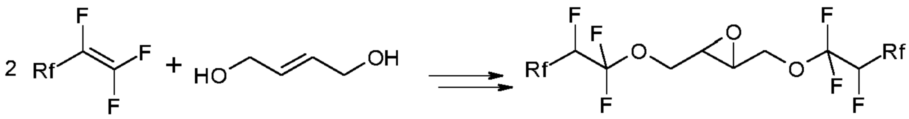

(IV) (VI) (II)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist.

**5.** Verfahren zur Herstellung von Verbindungen der Formel (III)

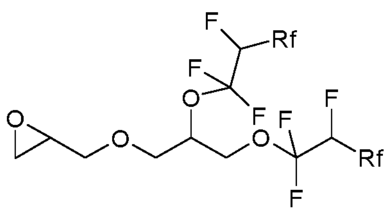

(III)

durch Umsetzen von Verbindungen der Formel (IV) mit Verbindungen der Formel (VII) zu Verbindungen der Formel (II)

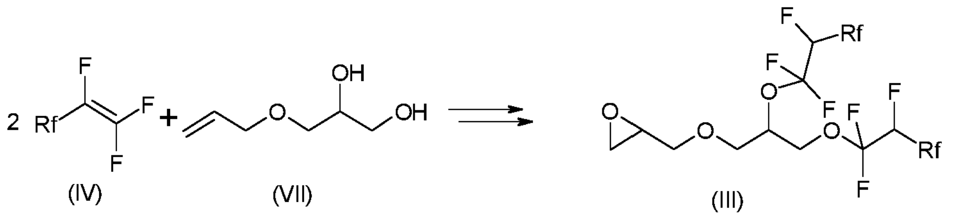

(IV) (VII) (III)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist.

**6.** Verfahren gemäß einem oder mehreren der Ansprüche 3 bis 5, **dadurch gekennzeichnet, dass** Rf eine Gruppe der Formel $CF_3-(CF_2)_{1-3}-$ oder $CF_3-(CF_2)_{1-3}-O-$ ist.

**7.** Verwendung von Verbindungen der Formel (I) zur Herstellung von Verbindungen der Formel (VIII)

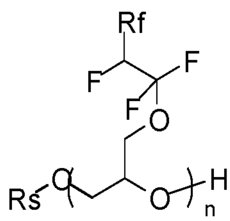
(VIII)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist,
Rs eine gegebenenfalls fluorierte Alkylgruppe ist und n gleich 5 bis 20 ist.

**8.** Verwendung von Verbindungen der Formel (II) zur Herstellung von Verbindungen der Formel (IX)

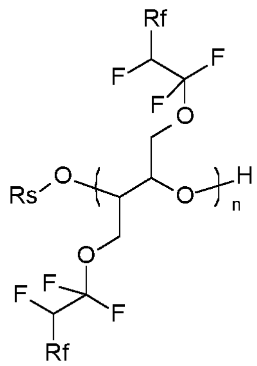

(IX)

wobei

Rf eine Gruppe der Formel $CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist,
Rs eine gegebenenfalls fluorierte Alkylgruppe ist und n gleich 5 bis 20 ist.

**9.** Verwendung von Verbindungen der Formel (III) zur Herstellung von Verbindungen der Formel (X)

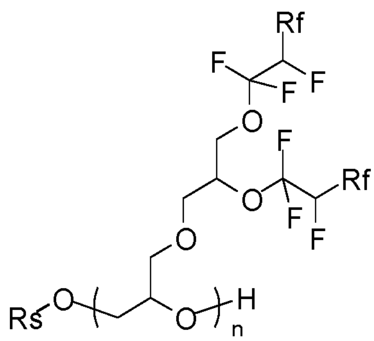

(X)

wobei

Rf eine Gruppe der Formel $CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist,
Rs eine gegebenenfalls fluorierte Alkylgruppe ist und n gleich 5 bis 20 ist.

**10.** Verbindungen der Formel (XI) bis (XVI)

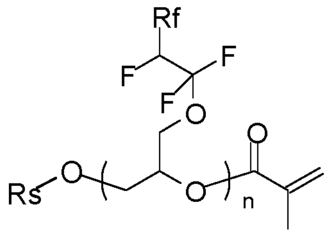
(XI)

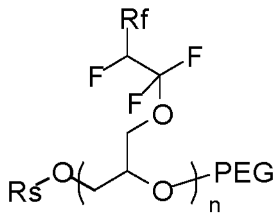
(XII)

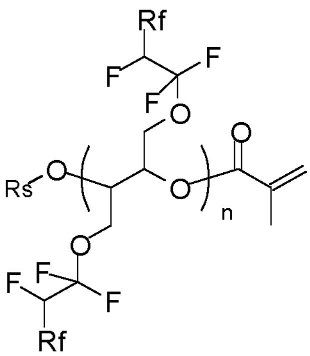
(XIII)

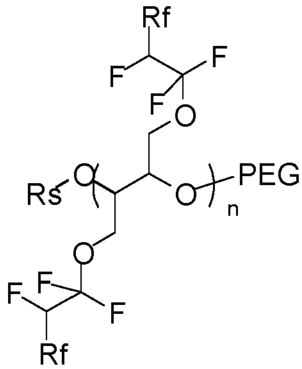
(XIV)

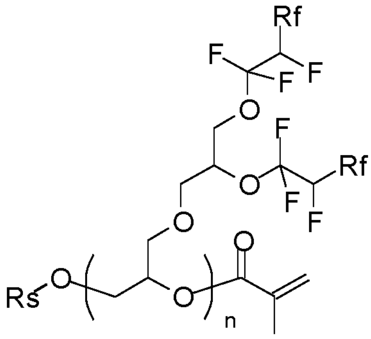
(XV)

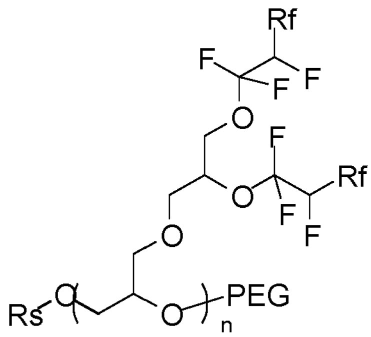
(XVI)

wobei

Rf eine Gruppe der Formel
$CF_3-(CF_2)_a-O_b-(CF_2)_c-O_d-$ ist,

wobei

a = 1, 2 oder 3,
b = 0 oder 1,
c = 0, 1, 2 oder 3 und
d = 0 oder 1 ist,
Rs eine gegebenenfalls fluorierte Alkylgruppe ist und n gleich 5 bis 20 ist.

**11.** Verbindungen gemäß Anspruch 10, **dadurch gekennzeichnet, dass** Rf eine Gruppe der Formel $CF_3-(CF_2)_{1-3}-$ oder $CF_3-(CF_2)_{1-3}-O-$ ist.

**12.** Verwendung von Verbindungen gemäß einem oder mehreren der Ansprüche 10 bis 11 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**13.** Mittel enthaltend eine oder mehrere Verbindungen gemäß einem oder mehreren der Ansprüche 10 bis 11 und einen für den jeweiligen Verwendungszweck geeigneten Träger sowie ggf. weitere spezifische Aktivstoffe.

**14.** Mittel nach Anspruch 13 **dadurch gekennzeichnet, dass** es sich bei dem Mittel um Farb- und Lackzubereitungen, Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

**15.** Beschichteter Gegenstand, dessen Beschichtung unter Verwendung von mindestens einer Verbindung gemäß einem oder mehreren der Ansprüche 10 bis 11 hergestellt wurde.

## Claims

**1.** Compounds of the formula (I), (II) or (III)

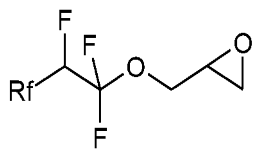

(I)

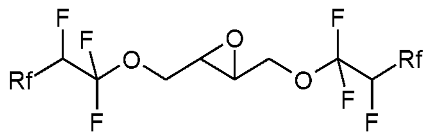

(II)

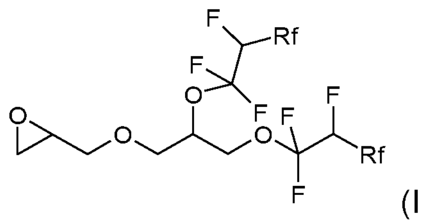

(III)

where

Rf is a group of the formula
$CF_3$-$(CF_2)_a$-$O_b$-$(CF_2)_c$-$O_d$-,

where

a = 1, 2 or 3,
b = 0 or 1,
c = 0, 1, 2 or 3 and
d=0 or 1.

**2.** Compounds according to Claim 1, **characterised in that** Rf is a group of the formula $CF_3$-$(CF_2)_{1-3}$- or $CF_3$-$(CF_2)_{1-3}$-O-.

**3.** Process for the preparation of compounds of the formula (I)

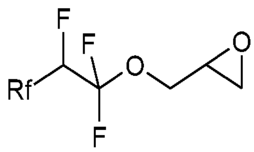

(I)

by reaction of compounds of the formula (IV) with glycidol (V) to give compounds of the formula (II)

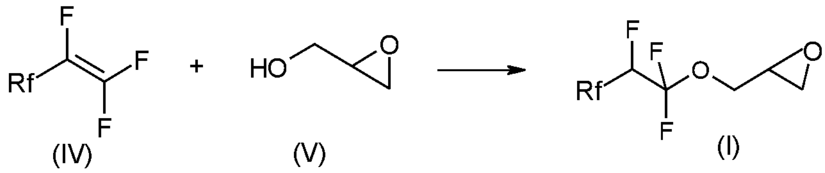

(IV) (V) (I)

where

Rf is a group of the formula
$CF_3-(CF_2)_a-O_b-(CF_2)_c-O_d-$,

where

a = 1, 2 or 3,
b = 0 or 1,
c = 0, 1, 2 or 3 and
d = 0 or 1.

**4.** Process for the preparation of compounds of the formula (II)

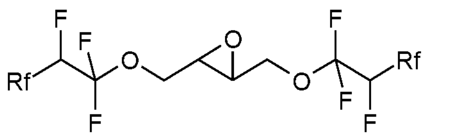

(II)

by reaction of compounds of the formula (IV) with compounds of the formula (VI) to give compounds of the formula (II)

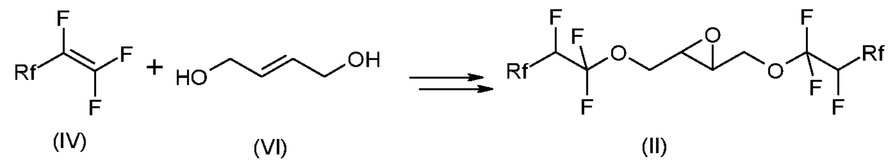

2

(IV) (VI) (II)

where

Rf is a group of the formula
$CF_3-(CF_2)_a-O_b-(CF_2)_c-O_d-$,

where

a = 1, 2 or 3,
b = 0 or 1,
c = 0, 1, 2 or 3 and
d = 0 or 1.

**5.** Process for the preparation of compounds of the formula (III)

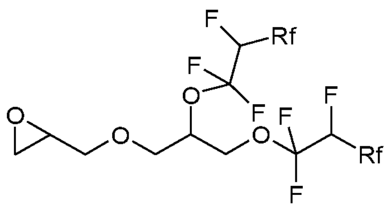

(III)

by reaction of compounds of the formula (IV) with compounds of the formula (VII) to give compounds of the formula (II)

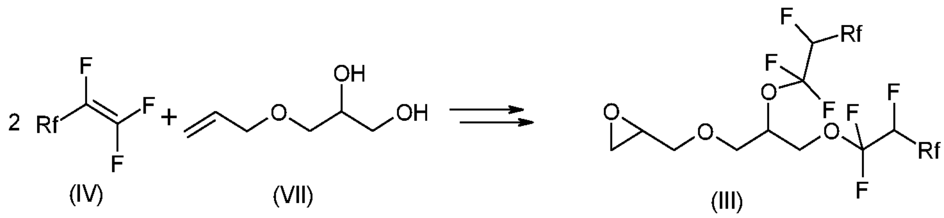

where

Rf is a group of the formula
$CF_3-(CF_2)_a-O_b-(CF2)c-Od-$,

where

a = 1, 2 or 3,
b = 0 or 1,
c = 0, 1, 2 or 3 and
d = 0 or 1.

**6.** Process according to one or more of Claims 3 to 5, **characterised in** Rf is a group of the formula $CF_3-(CF_2)_{1-3}-$ or $CF_3-(CF_2)_{1-3}-O-$.

**7.** Use of compounds of the formula (I) for the preparation of compounds of the formula (VIII)

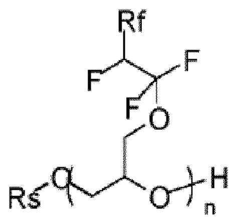

(VIII)

where

Rf is a group of the formula
$CF_3-(CF_2)_a-O_b-(CF2)c-Od-$,

where

a = 1, 2 or 3,
b = 0 or 1,
c = 0, 1, 2 or 3 and
d = 0 or 1,
Rs is an optionally fluorinated alkyl group and n is equal to 5 to 20.

**8.** Use of compounds of the formula (II) for the preparation of compounds of the formula (IX)

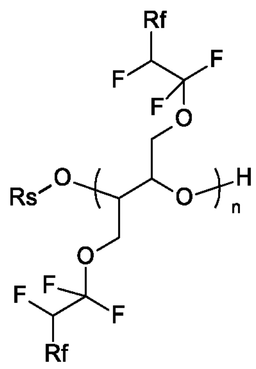

(IX)

where

Rf is a group of the formula
$CF_3-(CF_2)_a-O_b-(CF2)c-Od-$,

where

a = 1, 2 or 3,
b = 0 or 1,
c = 0, 1, 2 or 3 and
d = 0 or 1,
Rs is an optionally fluorinated alkyl group and n is equal to 5 to 20.

**9.** Use of compounds of the formula (III) for the preparation of compounds of the formula (X)

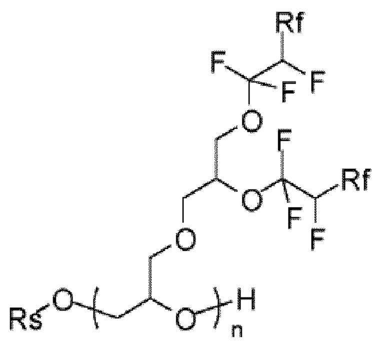

(X)

where

Rf is a group of the formula
$CF_3-(CF_2)_a-O_b-(CF2)c-Od-$,

where

a = 1, 2 or 3,
b = 0 or 1,
c = 0, 1, 2 or 3 and
d = 0 or 1,
Rs is an optionally fluorinated alkyl group and n is equal to 5 to 20.

**10.** Compounds of the formulae (XI) to (XVI)

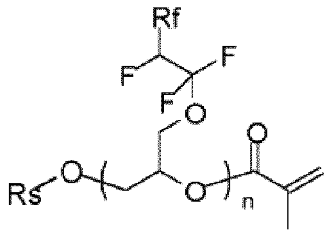

(XI)

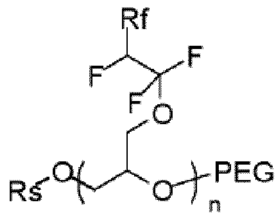

(XII)

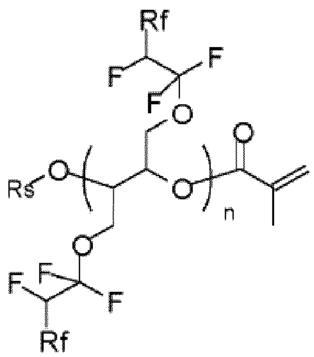

(XIII)

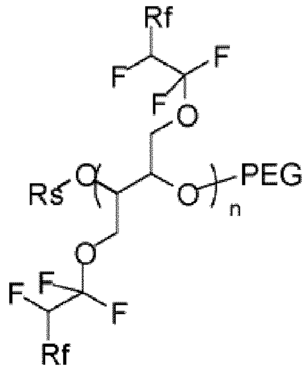

(XIV)

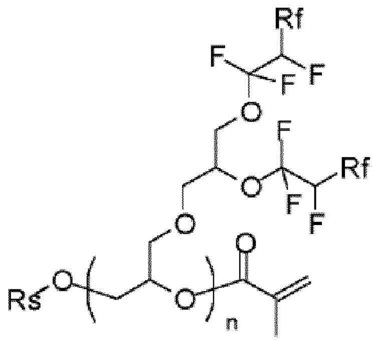

(XV)

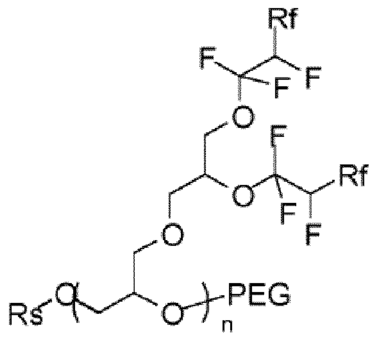

(XVI)

where

Rf is a group of the formula
$CF_3$-$(CF_2)_a$-$O_b$-(CF2)c-Od-,

where

a = 1, 2 or 3,
b = 0 or 1,
c = 0, 1, 2 or 3 and
d = 0 or 1,
Rs is an optionally fluorinated alkyl group and n is equal to 5 to 20.

**11.** Compounds according to Claim 10, **characterised in that** Rf is a group of the formula $CF_3$-$(CF_2)_{1\text{-}3}$- or $CF_3$-$(CF_2)_{1\text{-}3}$-O-.

**12.** Use of compounds according to one or more of Claims 10 to 11 as additives in paints, coatings, printing inks, protective coatings, special coatings in electronic or optical applications, photoresists, top antireflective coatings or bottom antireflective coatings, developer solutions and wash solutions and photoresists for photolithographic processes, cosmetic products, agrochemicals, floor polishes, photographic coatings or coatings of optical elements.

**13.** Composition comprising one or more compounds according to one or more of Claims 10 to 11 and a vehicle which is suitable for the respective application and optionally further specific active substances.

**14.** Composition according to Claim 13, **characterised in that** the composition is paint and coating preparations, fire-extinguishing compositions, lubricants, detergents and cleaning compositions, de-icers, developer solutions and wash solutions and photoresists for photolithographic processes, cosmetic products, agrochemicals, floor polishes or hydrophobicising compositions for textile finishing or glass treatment.

**15.** Coated article whose coating has been produced using at least one compound according to one or more of Claims 10 to 11.

**Revendications**

**1.** Composés de formule (I), (II) ou (III)

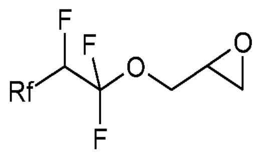

(I)

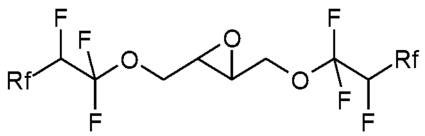

(II)

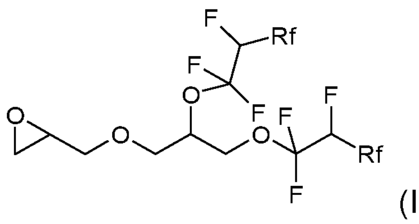

(III)

dans lesquels

Rf est un groupement de formule
CF3-(CF2)a-Ob-(CF2)c-Od-,

où

a = 1, 2 ou 3,
b = 0 ou 1,
c = 0, 1, 2 ou 3 et
d = 0 ou 1.

**2.** Composés selon la revendication 1, **caractérisés en ce que** Rf est un groupement de formule $CF_3-(CF_2)_{1-3}-$ ou $CF_3-(CF_2)_{1-3}-O-$.

**3.** Procédé de préparation de composés de formule (I)

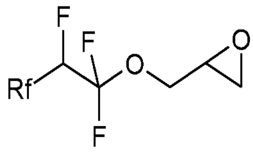

(I)

par la réaction de composés de formule (IV) avec du glycidol (V) pour conduire aux composés de formule (II)

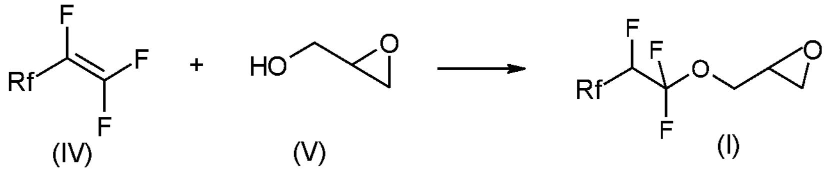

(IV) (V) (I)

dans lesquels

Rf est un groupement de formule
CF3-(CF2)a-Ob-(CF2)c-Od-,

où

a = 1, 2 ou 3,
b = 0 ou 1,
c = 0, 1, 2 ou 3 et
d = 0 ou 1.

**4.** Procédé de préparation de composés de formule (II)

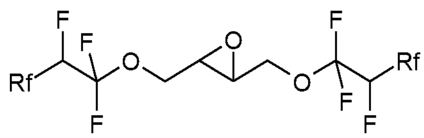

(II)

par la réaction de composés de formule (IV) avec des composés de formule (VI) pour conduire aux composés de formule (II)

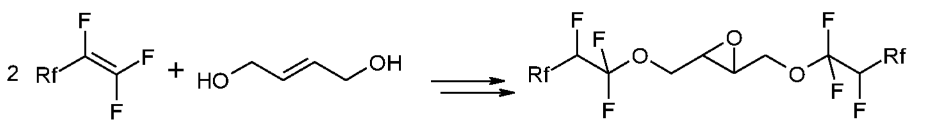

(IV) (VI) (II)

dans lesquels

Rf est un groupement de formule
CF3-(CF2)a-Ob-(CF2)c-Od-,

où

a = 1, 2 ou 3,
b = 0 ou 1,
c = 0, 1, 2 ou 3 et
d = 0 ou 1.

**5.** Procédé de préparation de composés de formule (III)

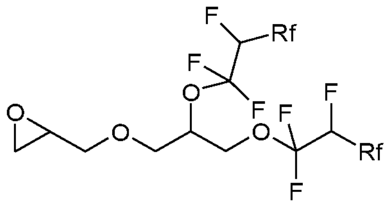

(III)

par la réaction de composés de formule (IV) avec des composés de formule (VII) pour conduire aux composés de formule (II)

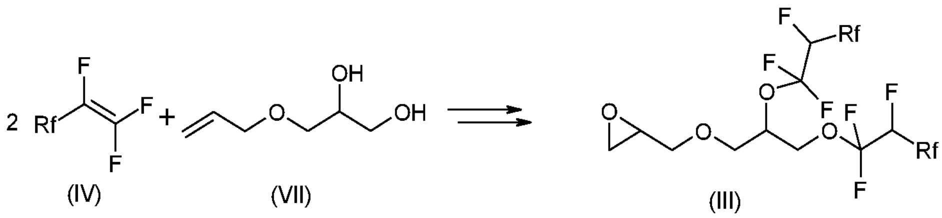

(IV) (VII) (III)

dans lesquels

Rf est un groupement de formule
$CF_3-(CF_2)_a-O_b-(CF_2)_c-O_d-$,

où

a = 1, 2 ou 3,
b = 0 ou 1,
c = 0, 1, 2 ou 3 et
d = 0 ou 1.

**6.** Procédé selon l'une ou plusieurs parmi les revendications 3 à 5, **caractérisé en ce que** Rf est un groupement de formule $CF_3-(CF_2)_{1-3}-$ ou $CF_3-(CF_2)_{1-3}-O-$.

**7.** Utilisation de composés de formule (I) pour la préparation de composés de formule (VIII)

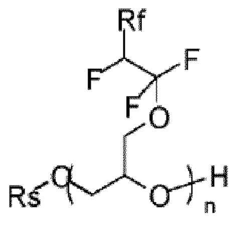

(VIII)

dans lesquels

Rf est un groupement de formule
$CF_3-(CF_2)_a-O_b-(CF_2)_c-O_d-$,

où

a = 1, 2 ou 3,
b = 0 ou 1,
c = 0, 1, 2 ou 3 et

d = 0 ou 1,
Rs est un groupement alkyle éventuellement fluoré et n est égal à de 5 à 20.

**8.** Utilisation de composés de formule (II) pour la préparation de composés de formule (IX)

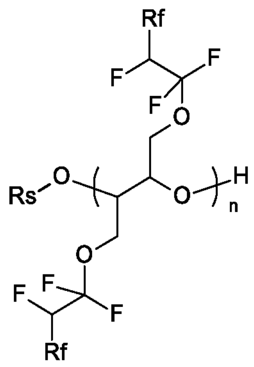

(IX)

dans lesquels

Rf est un groupement de formule
$CF_3-(CF_2)_a-O_b-(CF_2)_c-O_d-$,

où

a = 1, 2 ou 3,
b = 0 ou 1,
c = 0, 1, 2 ou 3 et
d = 0 ou 1,
Rs est un groupement alkyle éventuellement fluoré et n est égal à de 5 à 20.

**9.** Utilisation de composés de formule (III) pour la préparation de composés de formule (X)

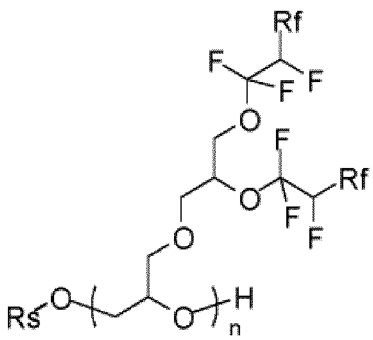

(X)

dans lesquels

Rf est un groupement de formule
$CF_3-(CF_2)_a-O_b-(CF_2)_c-O_d-$,

où

a = 1, 2 ou 3,
b = 0 ou 1,
c = 0, 1, 2 ou 3 et
d = 0 ou 1,

Rs est un groupement alkyle éventuellement fluoré et n est égal à de 5 à 20.

**10.** Composés de formules (XI) à (XVI)

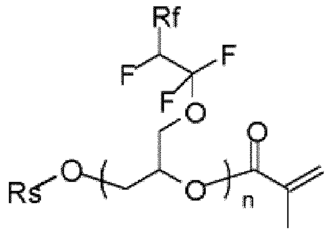
(XI)

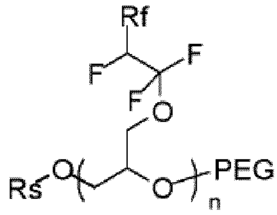
(XII)

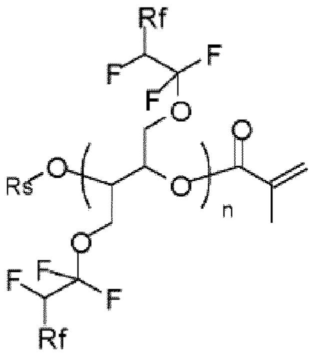
(XIII)

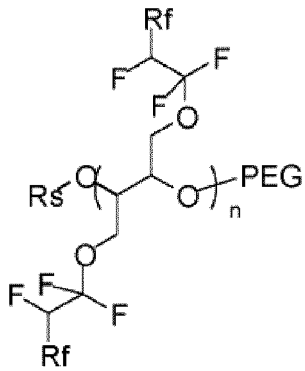
(XIV)

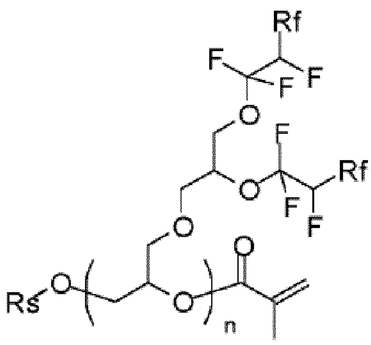
(XV)

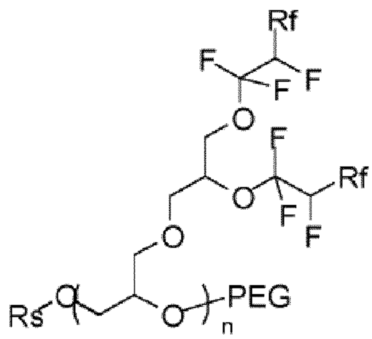
(XVI)

dans lesquels

Rf est un groupement de formule
$CF_3-(CF_2)_a-O_b-(CF_2)_c-O_d-$,

où

a = 1, 2 ou 3,
b = 0 ou 1,
c = 0, 1, 2 ou 3 et
d = 0 ou 1,
Rs est un groupement alkyle éventuellement fluoré et n est égal à de 5 à 20.

**11.** Composés selon la revendication 10, **caractérisés en ce que** Rf est un groupement de formule $CF_3-(CF_2)_{1-3}-$ ou $CF_3-(CF_2)_{1-3}-O-$.

**12.** Utilisation de composés selon l'une ou plusieurs parmi les revendications 10 à 11, comme additifs dans les peintures,

les revêtements, les encres d'impression, les revêtements protecteurs, les revêtements spéciaux dans les applications électroniques ou optiques, les résines photosensibles, les revêtements antireflets supérieurs ou les revêtements antireflets inférieurs, les solutions de révélateur et les solutions de lavage ainsi que les résines photosensibles pour les procédés photo-lithographiques, les produits cosmétiques, les substances agrochimiques, les encaustiques, les revêtements photographiques ou les revêtements d'éléments optiques.

**13.** Composition comprenant un ou plusieurs composés selon l'une ou plusieurs parmi les revendications 10 à 11 et un véhicule qui est convenable pour l'application respective, et éventuellement d'autres substances actives spécifiques.

**14.** Composition selon la revendication 13, **caractérisée en ce que** la composition est constituée de préparations de peinture et de revêtement, de compositions d'extinction d'incendies, de lubrifiants, de compositions de lavage et de détergents, de dégivreurs, de solutions de révélateur et de solutions de lavage ainsi que de résines photosensibles pour les procédés photo-lithographiques, de produits cosmétiques, de substances agrochimiques, d'encaustiques ou de compositions d'hydrophobie pour la finition des textiles ou le traitement du verre.

**15.** Article revêtu, dont le revêtement a été produit en utilisant au moins un composé selon l'une ou plusieurs parmi les revendications 10 à 11.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016096128 A1
- WO 2016069129 A1
- WO 2015124290 A1


### In der Beschreibung aufgeführte Nicht-Patentliteratur


- **SOLOV'EV et al.** *Zhurnal Obshchei Khimii,* 1991, vol. 61 (3), 673-679
- **J.D.LAZERTE ; R.J. KOSHAR.** *Journal of the American Chemical Society,* 1955, vol. 77, 910-914